# EUROPEAN PATENT SPECIFICATION

(11) **EP 4 180 329 B1**
(45) Date of publication and mention of the grant of the patent: **11.09.2024**
(21) Application number: 21207607.9
(22) Date of filing: 10.11.2021
(51) Int. Cl.: B64D 11/00, B64D 13/06, H01J 61/54, H01J 65/04, B64D 13/00

(54) **INTERIOR AIRCRAFT LIGHTING DEVICE, AIRCRAFT COMPRISING AN INTERIOR AIRCRAFT LIGHTING DEVICE AND METHOD OF STARTING AN INTERIOR AIRCRAFT LIGHTING DEVICE**
FLUGZEUGINNENBELEUCHTUNGSVORRICHTUNG, FLUGZEUG MIT DER FLUGZEUGINNENBELEUCHTUNGSVORRICHTUNG UND VERFAHREN ZUM STARTEN EINER FLUGZEUGINNENBELEUCHTUNGSVORRICHTUNG
DISPOSITIF D'ÉCLAIRAGE INTÉRIEUR D'AÉRONEF, AÉRONEF COMPRENANT UN DISPOSITIF D'ÉCLAIRAGE INTÉRIEUR D'AÉRONEF ET PROCÉDÉ DE DÉMARRAGE D'UN DISPOSITIF D'ÉCLAIRAGE INTÉRIEUR D'AÉRONEF

(43) Date of publication of application: 17.05.2023
(73) Proprietor: Goodrich Lighting Systems GmbH & Co. KG, 59557 Lippstadt (DE)
(72) Inventor: TRINSCHEK, Robert, 59067 Hamm (DE)
(74) Representative: Schmitt-Nilson Schraud Waibel Wohlfrom Patentanwälte Partnerschaft mbB

(56) References cited:
- WO-A1-2007/013602
- JP-B1- 6 919 753
- US-A1- 2003 025 451
- US-A1- 2010 225 254
- US-A1- 2018 079 528

## Description

The present invention is in the field of aircraft equipment. The present invention is in particular in the field of aircraft disinfection, more particularly in the field of aircraft disinfection using ultraviolet light. In the following, ultraviolet light will be referred to as "UV light".

For reducing the risk of distributing infectious diseases in an aircraft, it is desirable to regularly disinfect surfaces within the aircraft, which are routinely contacted by passengers and/or aircraft personnel. UV light may be used as a germicidal illumination for disinfecting the surfaces within an aircraft by irradiation.

US 2018/0079528 A1 discloses apparatuses and methods for use of an ultraviolet (UV) light source, such as in a cleaning device, in which ozone creation due to reaction of the UV light with oxygen in the air is reduced. An example method includes dispersing, by a gas outlet, oxygen-depleted gas over a UV light source, and directing UV light from the UV light source to pass through the oxygen-depleted gas onto an area. An example apparatus includes a UV light source to direct UV light onto an area, and a gas outlet to disperse oxygen-depleted gas over the UV light source, such that the UV light passes through the oxygen-depleted gas onto the area.

JP 6 919 753 B1 discloses an ultraviolet irradiation device that includes a plurality of excimer lamps with light-emitting tubes filled with discharge gases including rare gases. The plurality of excimer lamps includes a first excimer lamp in which a discharge gas is filled at a first filled gas pressure and a second excimer lamp in which a discharge gas is filled at a second filled gas pressure lower than the first filled gas pressure. The first excimer lamp is positioned at a position where at least a part of light emitted from the second excimer lamp can enter the first excimer lamp.

US 2010/0225254 A1 discloses a cold cathode lamp that includes a light-transmissive insulating tube; the first and second internal electrodes disposed inside the insulating tube; the first and second external electrodes disposed outside the insulating tube and connected to the first and second internal electrodes, respectively; the first and second insulating members covering the first and second external electrodes, respectively. It is possible to light up a plurality of cold cathode lamps that are connected in parallel to a power supply.

UV light sources, which may be employed for generating UV light, include gas discharge lamps. Gas discharge lamps comprise at least one excitable gas, which may generate electromagnetic radiation, in particular electromagnetic radiation including UV light, upon electric excitation. Usually, high electric voltages of several thousand volts are necessary for triggering a gas discharge reaction. In consequence, high voltage electric power supplies are needed for providing the high electric voltage needed for starting and operating gas discharge lamps, leading to high complexity, high cost, and high efforts for safety / electric isolation purposes.

It would be beneficial to provide an interior aircraft lighting device that is effective for disinfecting and that is easy to handle, in particular an interior aircraft lighting device that may have reduced electric voltages needed for generating the UV light employed for disinfection in an aircraft.

Exemplary embodiments of the invention include an interior aircraft lighting device in accordance with claim 1, an aircraft in accordance with claim 9, and a method of disinfecting at least one component or surface within an aircraft in accordance with claim 13. Further embodiments of the invention are given in the dependent claims. Exemplary embodiments of the invention include an interior aircraft lighting device comprises at least two discharge light modules. The at least two discharge light modules include at least one first discharge light module and at least one second discharge light module. Each discharge light module contains at least one excitable gas. The at least one excitable gas emits electromagnetic radiation, in particular UV light, pursuant to electric excitation. In particular, the excitable gas may emit electromagnetic radiation, when suitably triggered and when an electric field, suitable for maintaining a gas discharge reaction, is continuously applied. The at least one excitable gas may be a single gas or a mixture of two or more gases. The interior aircraft lighting device further comprises a plurality of electrodes, including at least two pairs of electrodes.

At least one pair of electrodes is assigned to each discharge light module for applying an electric field, extending between the two electrodes, to the at least one gas within the respective discharge light module. The electrodes may in particular be arranged outside the discharge light modules. The electrodes may further in particular be arranged outside the discharge light modules, but apply an electric field to the at least one gas contained within the respective discharge light modules.

Each pair of electrodes comprises two electrodes, which are spaced apart from each other along a longitudinal direction of the respective discharge light module. In consequence, the electric field, which is generated by applying an electric voltage to the two electrodes of a pair of electrodes, extends through the at least one gas, which is present between the two electrodes of the respective pair of electrodes.

The at least two pairs of electrodes include at least one first pair of electrodes, which is assigned to the at least one first discharge light module, and at least one second pair of electrodes, which is assigned to the at least one second discharge light module. In an interior aircraft lighting device according to exemplary embodiments of the invention, the distance between the two electrodes of the at least one first pair of electrodes, which is assigned to the at least one first discharge light module, is smaller than the distance between the two electrodes of the at least one second pair of electrodes, which is assigned to the at least one second discharge light module.

Since the distance between the two electrodes of the at least one first pair of electrodes is smaller than the distance between the two electrodes of the at least one second pair of electrodes, the minimum electric voltage for starting a gas discharge reaction between the two electrodes of the at least one first pair of electrodes is lower than the minimum electric voltage for starting a gas discharge reaction between the two electrodes of the at least one second pair of electrodes.

In consequence, a gas discharge reaction generating electromagnetic radiation, which in particular includes UV light, may be started between the two electrodes of the at least one first pair of electrodes by applying an electric voltage which is lower than the electric voltage which would be necessary for starting a gas discharge reaction between the two electrodes of the at least one second pair of electrodes, in which the distance between the two electrodes is larger than in the first pair of electrodes. This is because the electric field, which ultimately triggers the gas discharge reaction, is a function of the electric voltage between the electrodes and the distance between the electrodes, with a smaller distance between the electrodes resulting in a larger electric field for a given electric voltage.

After a first gas discharge reaction has been started between the two electrodes of the at least one first pair of electrodes, the electromagnetic radiation generated by said first gas discharge reaction, in particular the UV light portion of the electromagnetic radiation generated by said first gar discharge reaction, may excite the at least one gas between other electrodes, in particular the at least one gas which is present between the two electrodes of the at least one second pair of electrodes, which are assigned to the at least one second discharge light module.

Exciting the at least one gas with electromagnetic radiation reduces the strength of the electric field, which is required for starting a gas discharge reaction in said at least one gas. In other words, the electric voltage, which needs to be applied to the at least one second pair of electrodes for starting a gas discharge reaction between the two electrodes of said at least one second pair of electrodes, is reduced by exciting the at least one gas with electromagnetic radiation, in particular with electromagnetic radiation generated by the first gas discharge reaction.

In consequence, when the at least one gas between the two electrodes of the second discharge light module is excited by electromagnetic radiation, a second gas discharge reaction in the second discharge light module may be started by applying a lower voltage to the electrodes of the second pair of electrodes than in absence of electromagnetic radiation. The second gas discharge reaction in the second discharge light module may in particular be triggered by applying the same electric voltage, which has been applied to the two electrodes of the at least one first pair of electrodes for starting the first gas discharge reaction, to the at least one second pair of electrodes, although the second pair of electrodes has a larger distance therebetween than the first pair of electrodes.

As a result, gas discharge reactions in all discharge light modules of an interior aircraft lighting device according to exemplary embodiments of the invention may be triggered by applying a comparably low electric voltage, which is sufficient for triggering a gas discharge reaction between the at least one first pair of electrodes, assigned to the first discharge light module. In consequence, a power supply, which is capable of providing such a comparably low electric voltage, may be sufficient for operating the interior aircraft lighting device, and the complexity, costs, and/or isolation requirements of the power supply may be reduced.

In an embodiment, the discharge light modules may have a tubular shape extending in the longitudinal direction. The discharge light modules may have a circular or oval cross-section. Other shapes of the discharge light modules are possible as well.

In an embodiment, the at least two discharge light modules are supported by a common support, in particular by a common support plate. The common support plate may be a printed circuit board, in particular a printed circuit board comprising electric conductors for electrically coupling the electrodes with the electric power supply.

In an embodiment, the at least two discharge light modules are supported by separate supports, in particular by separate support plates, for example separate printed circuit boards. Using a plurality of separate supports for supporting the different discharge light modules may allow for a flexible arrangement of the discharge light modules in space. In such a configuration, the at least two discharge light modules may in particular be arranged independently of each other.

In an embodiment, the interior aircraft lighting device comprises a plurality, i.e. at least two, first discharge light modules; and/or the interior aircraft lighting device comprises a plurality, i.e. at least two, second discharge light modules.

Each of the first discharge light modules may comprise at least one first pair of electrodes, and each of the second discharge light modules may comprise at least one second pair of electrodes.

The distances between the two electrodes of the at least one first pair of electrodes are smaller than the distances between the two electrodes of the at least one second pair of electrodes. In other words, the first pairs of electrodes and the second pairs of electrodes may be defined by the distance between the electrodes of each of said pairs. In the context of the present application, the first pairs of electrodes may be defined as pairs of electrodes in which the distance between the two electrodes of each pair is smaller than a predefined distance, and the second pairs of electrodes may be defined as pairs of electrodes in which the distance between the two electrodes of each pair is larger than the predefined distance.

In an embodiment, the distances between the electrodes may be identical in all first pairs of electrodes, and the distances between the electrodes may be identical in all second pairs of electrodes, respectively.

In another embodiment, the distances between the two electrodes of all first pairs of electrodes are not identical, and/or the distances between the two electrodes of all second pairs of electrodes are not identical. The distances between the two electrodes of every first pair of electrodes, however, may be smaller than the predefined distance, and the distances between the two electrodes of every second pair of electrodes may be larger than the predefined distance, so that the distances between the two electrodes of all first pairs of electrodes are smaller than the distances between the two electrodes of all second pairs of electrodes.

In the context of the present application, a first discharge light module may be defined as a discharge light module to which at least one first pair of electrodes is assigned. Aa second discharge light module may be defined as a discharge light module to which no first pair of electrodes is assigned.

A plurality of second pairs of electrodes may be assigned to a second discharge light module. A plurality of pairs of electrodes including at least one first pair of electrodes may be assigned to a first discharge light module. In other words, any combination of first and second pairs of electrodes, including at least one first pair of electrodes, may be assigned to a first discharge light module.

In an embodiment, an odd number of electrodes may be assigned to a discharge light module. In such an embodiment, the electrodes assigned to a discharge light module may include a central electrode, which may be arranged between two outer electrodes along the longitudinal direction. In such a configuration, the central electrode may form one pair of electrodes with one of the outer electrodes and the central electrode may further form another pair of electrodes with the other one of the outer electrodes.

The distances between the central electrode and the two outer electrodes may be identical, so that the three electrodes form two similar pairs of electrodes, in which the distances between adjacent electrodes are substantially the same. Alternatively, the distance between the central electrode and a first one of the outer electrodes may differ from the distance between the central electrode and a second one of the outer electrodes. In such a configuration, the three electrodes form two different pairs of electrodes, in which the distances between the electrodes of each pair of electrodes are different.

The interior aircraft lighting device may comprise two, three, four, five, six, seven, eight, nine, ten or more first and/or second discharge light modules, respectively. Increasing the number of first and/or second discharge light modules may result in an increase of the total amount of electromagnetic radiation emitted by the interior aircraft lighting device.

In an embodiment, each discharge light module has a power capacity between 0.5 W an 2.0 W, in particular a power capacity between 1.0 W and 1.5 W.

In an embodiment, the at least two discharge light modules are arranged next to each other in a side-by-side arrangement such that electromagnetic radiation emitted by any of the at least two discharge light modules excites the at least one excitable gas in at least one other discharge light module, in particular the at least one excitable gas in at least one adjacent discharge light module.

In such a configuration, the electromagnetic radiation emitted by at least one discharge light module, in which a gas discharge reaction has been started, supports starting a gas discharge reaction in at least one other discharge light module. I.e., after a gas discharge reaction has been started between at least one first pair of electrodes of a first discharge light module, the electromagnetic radiation generated by said gas discharge reaction may excite the at least one gas between the electrodes of at least one other pair of electrodes, which may be arranged in a larger distance from each other than the first pair of electrodes, so that the at least one gas between said electrodes may be ignited by applying the same, relatively low voltage, which has been applied to the first pair of electrodes.

Said at least one other pair of electrodes may by arranged at the same discharge light module as the first pair of electrodes. I.e. a discharge light module may comprise at least two pairs of electrodes including a first pair of electrodes, which are arranged in a small first distance from each other, and at least one further pair of electrodes, comprising electrodes which are arranged in the first distance or in a larger second distance from each other. The further pair of electrodes may also be a second pair of electrodes, which is arranged at a different discharge light module, in particular at a second discharge light module.

The electromagnetic radiation emitted by the gas discharge reaction, which has been started between said other pair of electrodes, may excite at least one gas in at least one further discharge light module. The electromagnetic radiation emitted by the gas discharge reaction, which has been started in said at least one further discharge light module may excite at least one gas in yet another discharge light module and so on.

In consequence, gas discharge reactions in all discharge light modules may by activated in a cascade or chain reaction, wherein the start of the first gas discharge reaction is facilitated by a small distance between the electrodes and each gas discharge reaction, except for the first gas discharge reaction, is supported by electromagnetic radiation generated by at least one previously started gas discharge reaction.

In an embodiment, the discharge light modules are arranged in a parallel arrangement, i.e. in an arrangement, in which the longitudinal directions of the discharge light modules are oriented basically parallel to each other. The discharge light modules may be spaced apart from each other in a lateral direction, which is oriented laterally, in particular basically orthogonally, to the longitudinal direction.

Such a parallel arrangement of the discharge light modules may allow for a spatially compact arrangement, in which the discharge light modules occupy only a relatively small area of space, but which may allow for generating an emission of electromagnetic radiation having a high energy density. Such a parallel arrangement may further allow for efficiently exciting the gas in at least one discharge light module with electromagnetic radiation generated by a gas discharge reaction, which was started in at least one other discharge light module.

In an embodiment, the discharge light modules may have a length in the longitudinal direction of between 20 mm and 100 mm, in particular a length of between 40 mm and 80 mm, more particularly a length of between 50 mm and 70 mm.

In an embodiment, the discharge light modules may have a diameter in a transverse direction, which extends transversly, in particular orthogonally, to the longitudinal direction, of between 2 mm and 8 mm, in particular a diameter of between 3 mm and 7 mm, more particularly a diameter of between 4 mm and 6 mm.

In an embodiment, the first distance between the two electrodes of a first pair of electrodes is between 2 mm and 7 mm, in particular between 4 mm and 6 mm, more particularly about 5 mm.

In an embodiment, the second distance between the two electrodes of a second pair of electrodes is between 4 mm and 20 mm, in particular between 8 mm and 12 mm, more particularly about 10 mm. The second distance may in particular be at least 20% larger than the first distance, further in particular at least 50% larger than the first distance, yet further in particular at least twice as large as the first distance.

The above mentioned dimensions of the discharge light modules and the above mentioned distances between the electrodes of the pairs of electrodes have been found suitable for generating an effective emission of electromagnetic radiation, in particular UV light, which is well suited for disinfection.

In an embodiment, the electromagnetic radiation, which is emitted by the at least one gas, after a gas discharge reaction has been started, includes electromagnetic radiation in the range of UV light, in particular electromagnetic radiation in the range from 210 nm to 230 nm, more particularly electromagnetic radiation in the range from 215 nm to 225 nm, and even more particularly electromagnetic radiation in the range from 221 nm to 223 nm. Electromagnetic radiation / UV light in the above mentioned ranges is very well suited for disinfecting surfaces and other components by irradiating said surfaces and/or components with said electromagnetic radiation.

In an embodiment, the at least one gas in the discharge light modules includes or is a gas mixture comprising at least two gases, in particular xenon and crypto-chloride. Such a gas mixture has been found as very efficient for generating UV radiation, which is very well suited for disinfecting surfaces and other components, by starting a gas discharge reaction is said gas mixture.

In an embodiment, the interior aircraft lighting device further comprises an electric power supply for applying an electric voltage to each pair of electrodes. The electric power supply may be configured for applying an electric voltage between 1000 V and 5000 V.

The electric power supply may be a variable electric power supply, which may allow for varying the electric voltage applied to the electrodes. In particular, the electric power supply may be configured to supply a trigger voltage in start operation and a lower voltage in a steady state operation. The voltage for the steady state operation may be significantly below the trigger voltage, in particular less than 90%, further in particular less than 80% of the trigger voltage.

In an interior aircraft lighting device according to exemplary embodiments of the present invention, a reduced electric voltage may be sufficient for starting the gas discharge reactions between the electrodes. In consequence, an electric power supply providing an electric voltage in the above mentioned ranges may be sufficient for starting and maintaining the gas discharge reactions. As compared to previous approaches, the electric voltage for starting the gas discharge reactions may be reduced by 20% or 30% or 40% or 50% or even more. In other words, as compared to previous approaches, the electric voltage for starting the gas discharge reactions may be less than 80%, in particular less than 70%, further in particular less than 60%, yet further in particular less than 50% of the starting voltages of previous approaches.

In an embodiment, the method may include gradually increasing the electric voltage applied to the pairs of electrodes until a gas discharge reaction is started. This may allow for determining the minimum electric voltage, which is necessary for starting a gas discharge reaction of the at least one excitable gas, which is present between the electrodes. Such a method may prevent the gas discharge light modules from being started with an electric voltage which is higher than necessary. Exemplary embodiments of the invention also include an aircraft, in particular a passenger aircraft, comprising at least one interior aircraft lighting device according to an exemplary embodiment of the invention.

Exemplary embodiments of the invention further include a method of disinfecting at least one component and/or surface within an aircraft, in particular within a passenger aircraft, wherein the method includes: starting a gas discharge reaction in at least two discharge light modules of an interior aircraft lighting device according to an exemplary embodiment of the invention by applying an electric voltage to every pair of electrodes of the at least two discharge light modules and irradiating the at least one component or surface with electromagnetic radiation emitted by the at least two discharge light modules of the interior aircraft lighting device. The electric voltage, which is applied to the pairs of electrodes may in particular be an electric voltage between 1000 V and 5000 V.

In an embodiment, the aircraft comprises a lavatory and/or a galley, and the at least one interior aircraft lighting device is installed within the lavatory and/or within the galley of the aircraft for disinfecting components and/or surfaces within the lavatory and/or galley by irradiating said components and/or surfaces with electromagnetic radiation / UV light emitted by the at least one interior aircraft lighting device.

In an embodiment, the aircraft comprises at least one passenger seat. At least one interior aircraft lighting device according to an exemplary embodiment of the invention may be arranged within or next to the at least one passenger seat for irradiating at least a portion of the at least one passenger seat with electromagnetic radiation, in particular UV light, which is emitted by the at least one interior aircraft lighting device. Such a configuration may allow for disinfecting at least a portion of the at least one passenger seat with electromagnetic radiation emitted by the at least one interior aircraft lighting device.

In an embodiment, the aircraft comprises at least one passenger service unit arranged above the at least one passenger seat. At least one interior aircraft lighting device according to an exemplary embodiment of the invention may be arranged within or next to the at least one passenger service unit for irradiating at least a portion of the at least one passenger service unit with electromagnetic radiation, in particular UV light, which is emitted by the at least one interior aircraft lighting device. Such a configuration may allow for disinfecting at least a portion of the at least one passenger service unit with electromagnetic radiation emitted by the at least one interior aircraft lighting device.

Exemplary embodiments of the invention also include using an interior aircraft lighting device according to an exemplary embodiment of the invention for disinfecting at least one component and/or surface within an aircraft by irradiating the at least one component and/or surface with electromagnetic radiation, which is emitted by the at least two discharge light modules of the interior aircraft lighting device.

Further exemplary embodiments of the invention are described below with respect to the accompanying drawings, wherein:
Figure 1 depicts an aircraft, in particular an air plane, in accordance with an exemplary embodiment of the invention in a schematic side view.
Figure 2 depicts a schematic view of an overhead passenger service unit (PSU).
Figure 3 depicts a schematic cut-open view of an aircraft in accordance with an exemplary embodiment of the invention, showing a passenger cabin of the aircraft.
Figure 4 depicts a schematic view of an interior aircraft lighting device according to an exemplary embodiment of the invention.
Figure 5 depicts a schematic view of an interior aircraft lighting device according to a further exemplary embodiment of the invention.

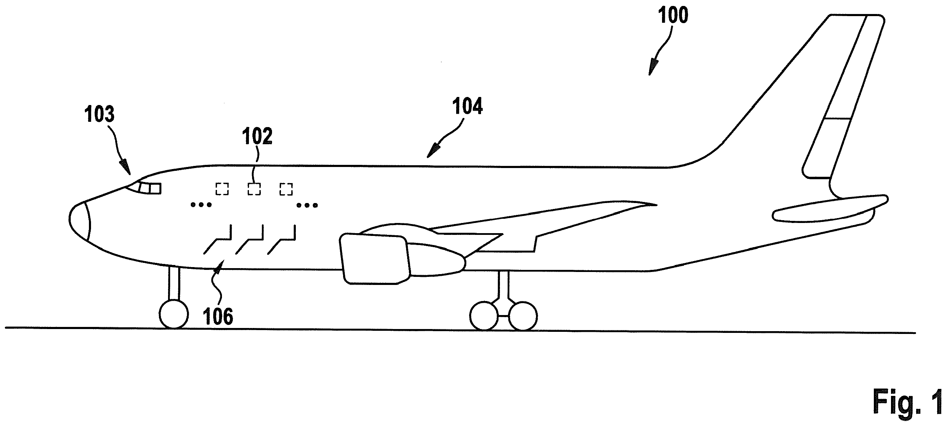

Figure 1 depicts an aircraft 100, in particular an air plane, in accordance with an exemplary embodiment of the invention in a schematic side view. In the exemplary embodiment shown in Figure 1, the aircraft 100 is a large passenger air plane, comprising a cockpit 103 and a passenger cabin 104 housing a plurality of passenger seats 106. The aircraft 100 may be a commercial passenger air plane, a private air plane, or a military aircraft. It is also possible that the interior aircraft lighting device and the method according to exemplary embodiments of the invention are implemented in a rotorcraft, such as a helicopter.

Passenger service units (PSU) 102 are arranged above the passenger seats 106.

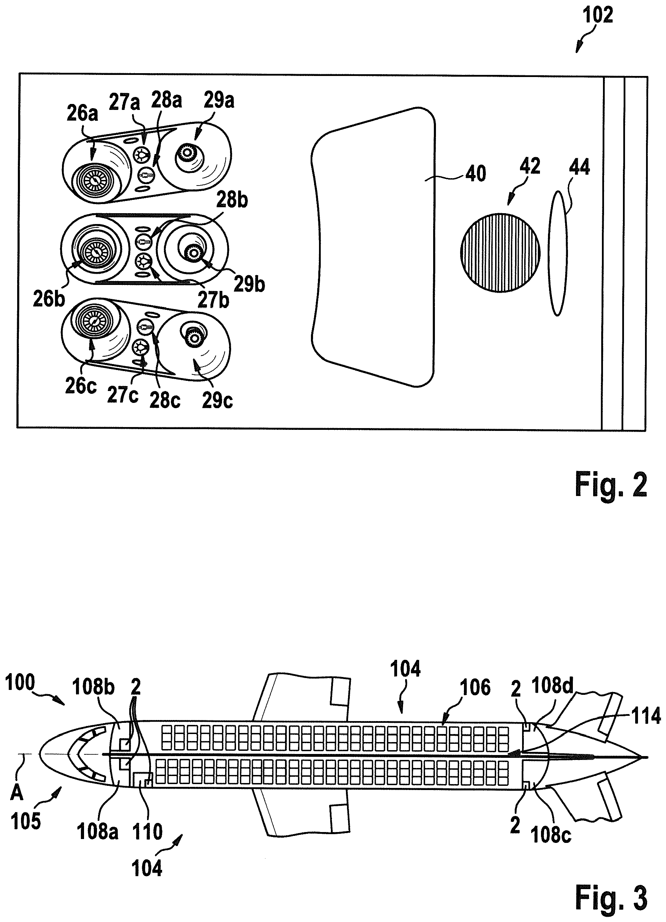

In an exemplary configuration, in which the aircraft 100 comprises six passenger seats 106 per row (cf. Figure 3, which will be discusses in more detail further below), each row of passenger seats 106 may have two passenger service units 102 associated therewith, one passenger service unit 102 assigned to the passenger seats 106 on the left side of a center aisle 114 and one passenger service unit 102 assigned to the passenger seats 106 on the right side of the center aisle 114.

Figure 2 depicts a schematic view of an overhead passenger service unit (PSU) 102, which is arranged above the passengers of a single passenger row, as it is seen from the side of a passenger sitting on a passenger seat 106 below the overhead passenger service unit 102.

On the side, which is shown to the left in Figure 2, the overhead passenger service unit 102 comprises a row of three adjustable reading lights 26a-26c, which are arranged next to each other.

Six electrical switches 27a-27c, 28a-28c are provided to the right side of the reading lights 26a-26c, a respective pair of two switches 27a-27c, 28a-28c next to each of the reading lights 26a-26c. One of the switches 27a-27c of each pair is configured for switching the adjacent reading light 26a-26c, and the second switch 28a-28c of each pair is configured for triggering a signal for calling cabin service personnel.

A row of three adjacent gaspers 29a-29c is provided next to the switches 27a-27c, 28a-28c.

Adjacent to the gaspers 29a-29c is a removable cover 40, which covers a cavity housing at least three oxygen masks (not shown). In the event of a pressure loss within the cabin, the removable cover 40 will open, the oxygen masks will drop out of the cavity, and each of the passengers, sitting below the overhead passenger service unit 102, may grasp one of the oxygen masks. The oxygen masks will be supplied with oxygen allowing the passengers to continue to breathe normally.

On the side opposite to the gaspers 29a-29c, a grid 42 is formed within the overhead passenger service unit 102. A loudspeaker (not shown), which may be used for delivering acoustic announcements to the passengers, is arranged behind said grid 42.

Next to the grid 42, there is a display panel 44, which may be configured for selectively showing a plurality of visual signs (not shown), such as "non smoking" or "fasten you seat belt". The display panel 44 may be illuminated from behind, in order to deliver visual information to the passengers sitting below the overhead passenger service unit 102.

Figure 3 depicts a schematic cut-open view of an aircraft 100 in accordance with an exemplary embodiment of the invention, depicting a passenger cabin 104 of the aircraft 100, also referred to as aircraft passenger cabin 104 herein.

The aircraft passenger cabin 104 is equipped with a plurality of passenger seats 106. The passenger seats 106 are arranged next to each other forming a plurality of passenger seat rows. Each passenger seat row comprises two groups of passenger seats 106, respectively including three passenger seats 106. The two groups of passenger seats 106 are separated from each other by a center aisle 114, extending along a longitudinal axis A of the aircraft 1.

The aircraft passenger cabin 104 is further equipped with four lavatories 108a-108d. In the exemplary configuration depicted in Figure 3, lavatories 108a-108d are provided at four locations within the aircraft passenger cabin 104. A first lavatory 108a is located at the front portside end of the aircraft passenger cabin 104, a second lavatory 108b is located at the front starboard end of the aircraft passenger cabin 104, a third lavatory 108c is located at the rear portside end of the aircraft passenger cabin 104, and a fourth lavatory 108d is located at the rear starboard end of the aircraft passenger cabin 104. Additionally or alternatively, lavatories 108a-108d may be provided at other locations of the aircraft passenger cabin 104 as well.

The aircraft passenger cabin 104 is further equipped with a galley 110, in order to allow for preparing meals and drinks for the passengers.

At least one of the lavatories 108a-108d and the galley 110 is provided with an interior aircraft lighting device 2 according to an exemplary embodiment of the invention.

In the exemplary embodiment depicted in Figure 3, each lavatory 108a-108d and the galley 110 are provided with an interior aircraft lighting device 2 for generating UV radiation, respectively. However, exemplary embodiments of the invention also include aircraft 100 in which only one or any subset of the lavatories 108a-108d and the galley 110 are provided with an interior aircraft lighting device 2 generating UV radiation.

Although not explicitly depicted in Figure 3, interior aircraft lighting devices 2 for generating UV radiation according to exemplary embodiments of the invention may also be provided next to the passenger service units 102 (cf. Figures 1 and 2) for disinfecting said passenger service units 102 by irradiating the passenger service units 102 with UV light.

Interior aircraft lighting devices 2 according to exemplary embodiments of the invention may also be provided within the passenger cabin 104 for irradiating and disinfecting the passenger seats 106 located under the passenger service units 102. The interior aircraft lighting devices 2 may, for example, be integrated into the passenger service units 102 or arranged next to the passenger service units 102.

interior aircraft lighting devices 2 according to exemplary embodiments of the invention may also be provided within the cockpit 103 of the aircraft 100 for disinfecting surfaces touched by the pilots.

As UV light may be harmful to humans, in particular to the human eye, the interior aircraft lighting devices 2 according to exemplary embodiments of the invention, provided within the passenger cabin 104 and/or within the cockpit 103 of the aircraft 100, may be activated only after the passengers and crew have disembarked after the flight, so that no humans are present within the aircraft 100.

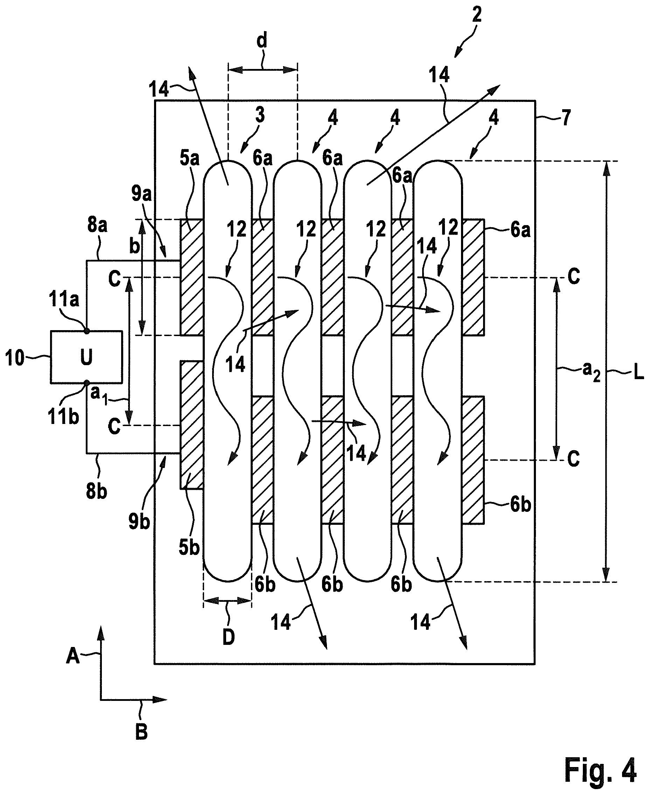

Interior aircraft lighting devices 2 according to exemplary embodiments of the invention, located in the lavatories 108a-108d, however, may also be activated during flight, when the lavatories 108a-108d are not occupied and the doors of the lavatories 108a-108d are closed, so that no or only very small amounts of UV light can exit the lavatories 108a-108d. interior aircraft lighting devices 2 located within the lavatories 108a-108d may, for example, be activated in regular time intervals or after a predefined number of passengers have used the respective lavatory 108a-108d, in order to ensure hygienic conditions within the lavatories during the flight. Figure 4 depicts a schematic view of an interior aircraft lighting device 2 according to an exemplary embodiment of the invention.

The interior aircraft lighting device 2 comprises at least two discharge light modules 3, 4, in particular four discharge light modules 3, 4, which are arranged in a side-by-side arrangement next to each other. The number of discharge light modules 3, 4 depicted in Figure 4 is only exemplary. The skilled person understands that an interior aircraft lighting device 2 according to an exemplary embodiment of the invention may comprise only two discharge light modules 3, 4 or any number of discharge light modules 3, 4, which is larger than two.

In the embodiment depicted in Figure 4, each of the four discharge light modules 3, 4 has a tubular shape. Each of the four discharge light modules 3, 4 has a length L in a longitudinal direction A of between 20 mm and 100 mm, in particular a length L of between 40 mm and 80 mm, more particularly a length L of between 50 mm and 70 mm. Each of the four discharge light modules 3, 4 has a diameter D of between 2 mm and 8 mm, in particular a diameter D of between 3 mm and 7 mm, more particularly a diameter D of between 4 mm and 6 mm.

The distances d between the discharge light modules 3, 4 in a lateral direction B, which is oriented orthogonally to the longitudinal direction A, are between 4 mm and 10 mm, in particular between 5 mm and 9 mm, more particularly between 6 mm and 8 mm.

The discharge light modules 3, 4 are made of glass or a similar material which is light transmissive and gas tight. Each discharge light module 3, 4 contains at least one excitable gas, in particular a mixture of at least two gases, which emits electromagnetic radiation 14 when an electric field having a sufficient strength is applied to the respective discharge light module 3, 4, so that the electric field passes through the at least one excitable gas.

In order to allow for applying such an electric field to the at least one excitable gas within the discharge light modules 3, 4, the interior aircraft lighting device 2 comprises a plurality of electrodes 5a, 5b, 6a, 6b. A pair of electrodes 5a, 5b, 6a, 6b is assigned to and arranged next to each discharge light module 3, 4, respectively. The electrodes 5a, 5b, 6a, 6b are located outside the respective discharge light modules 3, 4.

The discharge light modules 3, 4 and the electrodes 5a, 5b, 6a, 6b may be arranged on a common support 7, in particular a common support plate 7, such as a printed circuit board 7.

In alternative configurations, the discharge light modules 3, 4 and the electrodes 5a, 5b, 6a, 6b may be supported by different supports 7, in particular by multiple support plates or circuit boards 7. Such a configuration may allow for a more flexible arrangement of the discharge light modules 3, 4.

For supplying electric power to the electrodes 5a, 5b, 6a, 6b, the electrodes 5a, 5b, 6a, 6b are electrically coupled to an electric power supply 10 via electrical lines 8. Electric conducting paths 9a, 9b may be formed on and/or within the support 7 for electrically coupling the electrodes 5a, 5b, 6a, 6b to the electric power supply 10.

Each pair of electrodes 5a, 5b, 6a, 6b comprises a first electrode 5a, 6a and a second electrode 5b, 6b, respectively. The first electrodes 5a, 6a of all pairs of electrodes 5a, 5b, 6a, 6b are coupled to a first pole 11a of the electric power supply 10, and the second electrodes 5b, 6b of each pair of electrodes 5a, 5b, 6a, 6b are coupled to a second pole 11b of the electric power supply 10. In other words, the pairs of electrodes 5a, 5b, 6a, 6b are coupled in parallel to the electric power supply 10. In consequence, the electric power supply 10 applies the same voltage U to all pairs of electrodes 5a, 5b, 6a, 6b.

The electric power supply 10 is a high voltage power supply 10, which is capable of and configured for applying an electrical voltage of between 1000 V and 5000 V to the electrodes. The electric power supply 10 may be a variable electric power supply 10, which allows for varying the electric voltage U applied to the electrodes 5a, 5b, 6a, 6b.

In the embodiment depicted in Figure 4, each electrode 5a, 5b, 6a, 6b has a longitudinal extension b, i.e. an extension in the longitudinal direction A, of between 2 mm and 5 mm, in particular a longitudinal extension b of between 3 mm and 4 mm.

The discharge light modules 3, 4 include a first discharge light module 3, and a plurality, in particular three, second discharge light modules 4. A first pair of electrodes 5a, 5b is assigned to the first discharge light modules 3. A second pair of electrodes 6a, 6b is assigned to each of the second discharge light modules 4, respectively.

The two electrodes 5a, 5b, 6a, 6b of each pair of electrodes 5a, 5b, 6a, 6b are arranged in a distance a₁, a₂ from each other along the longitudinal direction A. The distances a₁, a₂ between the two electrodes 5a, 5b, 6a, 6b of each pair are measured between the centers C of the electrodes 5a, 5b, 6a, 6b along the longitudinal direction.

A first distance a₁ between the electrodes 5a, 5b of the first pair of electrodes 5a, 5b, which are assigned to the first discharge light module 3, is smaller than a second distance a₂ between the electrodes 6a, 6b of the second pairs of electrodes 6a, 6b, which are assigned to the second discharge light modules 4.

The first distance a₁ between the electrodes 5a, 5b of the first pair of electrodes 5a, 5b may be between 3 mm and 7 mm, in particular between 4 mm and 6 mm, more particularly between 4,5 mm and 5,5 mm. The second distance a₂ between the electrodes 6a, 6b of the second pairs of electrodes 6a, 6b may be between 6 mm and 14 mm, in particular between 8 mm and 12 mm, more particularly between 9,5 mm and 10 mm.

An electric voltage U, which exceeds a minimum ignition voltage U_{ign}, may be applied to the two electrodes 5a, 5b, 6a, 6b of a pair of electrodes 5a, 5b, 6a, 6b for starting a gas discharge reaction, generating a light bow 12 within the at least one gas contained in the associated discharge light module 3, 4. The ignition voltage U_{ign} is defined by the properties of the at least one gas within the discharge light modules 3, 4, and by the distance a₁, a₂ between the two electrodes 5a, 5b, 6a, 6b.

In a lighting device 2 according to an exemplary embodiment of the invention, as it is schematically depicted in Figure 4, the distance a₁ between the electrodes 5a, 5b of the first pair of electrodes 5a, 5b is smaller than the distances a₂ between the electrodes 6a, 6b of the second pairs of electrodes 6a, 6b. In consequence, a first minimum ignition voltage U_{ign1}, which is necessary for starting a gas discharge reaction generating a light bow 12 emitting electromagnetic radiation 14, in particular electromagnetic radiation 14 in the range of UV light, between the first electrodes 5a, 5b, which are assigned to the first discharge light module 3, is lower than a second minimum ignition voltage U_{ign2}, which is necessary for starting a gas discharge reaction between the second electrodes 6a, 6b, which are assigned to the second discharge light modules 4.

In other words, a gas discharge reaction, generating a light bow 12 emitting electromagnetic radiation 14, can be started within the first discharge light module 3 by applying a first voltage U₁ ≥ U_{ign1} to the first pair of electrodes 5a, 5a, wherein the first minimum ignition voltage U_{ign1} is lower than the second minimum ignition voltage U_{ign2}, which would be necessary for starting a gas discharge reaction between each of the second pairs of electrodes 6a, 6b, which are assigned to the second discharge light modules 4. Thus, when a first voltage U_{ign1} ≤ U₁ ≤ U_{ign2} is applied to all electrodes 5a, 5b, 6a, 6b of the interior aircraft lighting device 2, a gas discharge reaction is started only between the first electrodes 5a, 5b assigned to the first discharge light module 3, as the first voltage U₁ is not sufficient for starting a gas discharge reaction between the second electrodes 6a, 6b assigned to the second discharge light module 6.

However, when the at least one gas within a discharge light module 3, 4 is irradiated with electromagnetic radiation 14, in particular with electromagnetic radiation 14 including UV light, the gas molecules of the at least one gas are excited. As a result, the minimum ignition voltage U_{ign}, which needs to be applied to the electrodes 5a, 5b, 6a, 6b for starting a gas discharge reaction, generating a light bow 12 within the at least one gas, is reduced.

After a gas discharge reaction in the first discharge light module 3 has been started, as it has been described before, a portion of the electromagnetic radiation 14, which is emitted by the light bow 12, generated by said gas discharge reaction, irradiates and excites molecules of the at least one gas within at least one of the second discharge light modules 4. The electromagnetic radiation 14 in particular irradiates and excites the gas molecules of the at least one gas with the second discharge light module 4, which is arranged next to the first discharge light module 3. This excitation of the gas molecules reduces the voltage U_{ign}, which is necessary for starting a gas discharge reaction within said second discharge light module 4. In consequence, a gas discharge reaction within at least one second discharge light module 4, in particular within the second discharge light module 4, which is arranged next to the first discharge light module 3, may be started by applying the first voltage U₁ to the second part of electrodes 6a, 6b, which are assigned to said second discharge light module 4, as well.

The electromagnetic radiation 14, emitted by the light bow 12 generated by the gas discharge reaction within said at least one second discharge light module 4, will excite the gas molecules in at least one further second discharge light modules 4. This excitation may allow for starting an additional gas discharge reaction within at least one further second discharge light modules 4, without increasing the voltage U applied to the electrodes 5a, 5b, 6a, 6b beyond the first voltage U₁, which was applied to the first pair of electrodes 5a, 5b.

Thus, after a first gas discharge reaction has been started in the first discharge light module 3 by applying the first voltage U₁ to the first pair of electrodes 5a, 5b, further gas discharge reactions in additional second discharge light modules 4 may be started by applying the same, relatively low, first voltage U₁ to the electrodes 6a, 6b, assigned to said second discharge light modules 4, in a cascade or chain reaction, in which the gas molecules within at least one additional discharge light module 4 are excited by the electromagnetic radiation 14 emitted by the previously started discharge light modules 3, 4.

This cascade or chain reaction may allow for starting gas discharge reactions in all discharge light modules 4 by applying the first voltage U₁ to all pairs of electrodes 5a, 5b, 6a, 6b. The first voltage U₁ is lower than the second ignition voltage U_{ign2}, which would be necessary for starting gas discharge reactions in the second discharge light modules 4 having electrodes 6a, 6b, which are spaced apart at the larger distance a₂ from each other, if the gas molecules within the second discharge light modules 4 would not be excited by electromagnetic radiation 14.

As a result, the gas discharge reactions within all discharge light modules 3, 4 may be ignited by applying the lower first voltage U₁ to the electrodes 5a, 5b, 6a, 6b. Thus, there is no need for applying a higher second voltage U₂ ≥ U_{ign2} to any of the pairs electrodes 5a, 5b, 6a, 6b for starting the gas discharge reactions. In consequence, an interior aircraft lighting device 2 according to an exemplary embodiment of the invention, as it is depicted in Figure 4, may be ignited completely by applying the first voltage U₁ which is lower than the second ignition voltage U_{ign2}.

After all discharge light modules 3, 4 have been ignited, the power supply 10 may lower its output voltage to a voltage level sufficient for maintaining the emission of electromagnetic radiation from the discharge light modules 3, 4. In other words, the power supply 10 may lower its output voltage from a trigger voltage level, as described above, to a steady state operation voltage level. The voltage level for steady state operation may be below U_{ign1}.

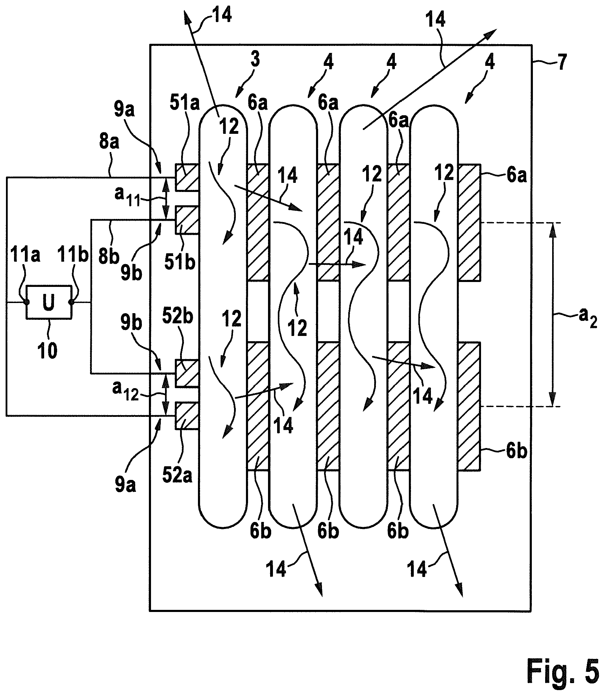

Figure 5 depicts a schematic view of an interior aircraft lighting device 2 according to another exemplary embodiment of the invention.

The components of the interior aircraft lighting device 2 depicted in Figure 5, which are analogous to the components of the interior aircraft lighting device 2 depicted in Figure 4, are denoted with the same reference signs, and the analogous features will not be discussed in detail again. Reference is made to their description above.

The interior aircraft lighting device 2, schematically depicted in Figure 5, differs from the interior aircraft lighting device 2, which is schematically depicted in Figure 4, in that in the embodiment shown in Figure 5, two pairs of electrodes 51a, 51b, 52a, 52b, in particular two first pairs of electrodes 51a, 51b, 52a, 52b, are assigned to the first discharge light module 3. In each of the two first pairs of electrodes 51a, 51b, 52a, 52b, the electrodes 51a, 51b, 52a, 52b are spaced apart from each other in a distance a₁₁, a₁₂, respectively, which is smaller then the second distance a₂ between the two electrodes 6a, 6b, assigned to the second discharge light modules 4.

Each of the two first pairs of electrodes 51a, 51b, 52a, 52b comprises a first electrode 51a, 52a, which is electrically coupled to the first pole 11a of the electric power supply 10, and a second electrode 51b, 52b, which is electrically coupled to the second pole 11b of the electric power supply 10.

In consequence, gas discharge reactions, generating a light bow 12 between the two electrodes 51a, 51b, 52a, 52b of each first pair of electrodes 51a, 51b, 52a, 52b, may be started by applying a first voltage U₁ ≥ U_{ign1} to the electrodes 51a, 51b, 52a, 52b of the first pairs of electrodes 51a, 51b, 52a, 52b, respectively.

By starting two gas discharge reactions within the first discharge light module 3, two light bows 12 are generated. The generation of an additional light bow 12 results in the emission of additional electromagnetic radiation 14 from the first discharge light module 3. As a result, the amount of electromagnetic radiation 14, in particular electromagnetic radiation 14 including UV light, emitted from the first discharge light module 3 and, in consequence, also the amount of electromagnetic radiation 14 emitted from the interior aircraft lighting device 2, are increased. This results in an enhanced efficiency of the interior aircraft lighting device 2.

Similar to the embodiment depicted in Figure 4, the electromagnetic radiation 14, emitted by the light bows 12 generated in the first discharge light module 3, excites gas molecules of the at least one gas within at least one of the second discharge light modules 4. This starts a cascade or chain reaction, which results in sequentially starting gas discharge reactions in all discharge light modules 3, 4, as it has been described before with respect to the embodiment depicted in Figure 4.

In the exemplary embodiment depicted in Figure 5, the distances a₁₁, a₁₂ between the two electrodes 51a, 51b, 52a, 52b of the two first pairs of electrodes 51a, 51b, 52a, 52b are identical. This, however, is only an exemplary configuration.

In an alternative configuration, which is not explicitly depicted in the figures, but which may also form an embodiment of the present invention, the first discharge light module 3 may be equipped with two pairs of electrodes 51a, 51b, 52a, 52b, wherein the distances a₁₁, a₁₂ between the electrodes 51a, 51b, 52a, 52b are different in each pair of electrodes 51a, 51b, 52a, 52b (a₁₁ ≠ a₁₂).

In particular, the distance a₁₁ of only one of the two pairs of electrodes 51a, 51b, 52a, 52b may be small enough for allowing a gas discharge reaction to be started by applying the relatively low voltage U_{ign1} to the electrodes 51a, 51b of said pair of electrodes 51a, 51b. The electromagnetic radiation 14, which is emitted by the light bow 12 generated by said gas discharge reaction, will support the starting of a further gas discharge reaction between the electrodes 52a, 52b of the other pair of electrodes 52a, 52b, which are provided at the first discharge light module 3, even if the distance a₁₂ between the electrodes 52a, 52b of said other pair of electrodes 52a, 52b is larger and would require a higher voltage U ≥ U_{ign1} to be applied to the electrodes 52a, 52b for starting a gas discharge reaction in the absence of electromagnetic radiation 14.

Increasing the distance a₁₂ between the electrodes 51a, 51b, 52a, 52b of a pair of electrodes 51a, 51b, 52a, 52b increases the length of the light bow 12, which is generated by the gas discharge reaction. In consequence, increasing the distance a₁₂ between the electrodes 51a, 51b, 52a, 52b increases the amount of electromagnetic radiation 14 emitted by the first discharge light module 3.

It therefore might be beneficial to arrange only the electrodes 51a, 51b of a single first pair of electrodes 51a, 51b in a first (lower) distance from each other, in order to allow for starting a gas discharge reaction between these electrodes 51a, 51b by applying a first (lower) voltage U₁ ≥ U_{ign1} to these electrodes 51a, 51b, and to arrange the electrodes 52a, 52b, 6a, 6b of all other pairs of electrodes 52a, 52b, 6a, 6b in a larger distance a₂ > a₁ from each other, in order to increase the total amount of electromagnetic radiation 14, which is emitted by the interior aircraft lighting device 2.

In the exemplary embodiments depicted in Figures 4 and 5, the electrodes 6a, 6b of all second pairs of electrodes 6a, 6b are arranged in the same distance a₂ from each other. Although it is not explicitly shown in the figures, it is also possible that the distances a₂ between the electrodes 6a, 6b of the second pairs of electrodes 6a, 6b are not the same in all second pairs of electrodes 6a, 6b. Instead, there may be at least one second pair of electrodes 6a, 6b, in which the distance a₂ between the electrodes 6a, 6b is different.

In such a configuration, it is possible that the distances a₂ between the electrodes 6a, 6b of the second pairs of electrodes 6a, 6b vary between the different discharge light modules 4. Alternatively or additionally, it is possible that two or more second pairs of electrodes 6a, 6b are assigned to at least some of the second discharge light modules 4. In such a configuration, the distances a₂ between the electrodes 6a, 6b of said second pairs of electrodes 6a, 6b, which are assigned to the same second discharge light module 4, may be indentical or different.

While the invention has been described with reference to exemplary embodiments, it will be understood by those skilled in the art that various changes may be made for elements thereof without departing from the scope of the invention as defined by the appended claims. In addition, many modifications may be made to adapt a particular situation or material to the teachings of the invention without departing from the scope thereof. Therefore, it is intended that the invention not be limited to the particular embodiment disclosed, but that the invention will include all embodiments falling within the scope of the appended claims.

## Claims

1. Interior aircraft lighting device (2), comprising:
at least two discharge light modules (3, 4), including at least one first discharge light module (3) and at least one second discharge light module (4), wherein each discharge light module (3, 4) contains at least one excitable gas, which emits electromagnetic radiation (14) pursuant to electric excitation;
multiple electrodes (5a, 5b, 6a, 6b, 51a, 51b, 52a, 52b), wherein at least one
pair of electrodes (5a, 5b, 6a, 6b, 51a, 51b, 52a, 52b) of the multiple electrodes (5a, 5b, 6a, 6b, 51 a, 51 b, 52a, 52b) is assigned to each discharge light module (3, 4) for applying an electric field to the at least one excitable gas within the respective discharge light module (3, 4);
wherein the two electrodes (5a, 5b, 6a, 6b, 51a, 51b, 52a, 52b) of each pair of electrodes (5a, 5b, 6a, 6b, 51a, 51b, 52a, 52b) are spaced apart from each other along a longitudinal direction (A) of the respective discharge light module (3, 4);
wherein the pairs of electrodes (5a, 5b, 6a, 6b, 51a, 51b, 52a, 52b) include at least one first pair of electrodes (5a, 5b, 51a, 51b, 52a, 52b), which is assigned to the at least one first discharge light module (3);
**characterized in that** the pairs of electrodes (5a, 5b, 6a, 6b, 51 a, 51 b, 52a, 52b) include
at least one second pair of electrodes (6a, 6b), which is assigned to the at least one second discharge light module (4); and
wherein a distance (a₁, a₁₁, a₁₂) between the two electrodes (5a, 5b, 51a, 51b, 52a, 52b) of the at least one first pair of electrodes (5a, 5b, 51a, 51b, 52a, 52b) is smaller than a distance (a₂) between the two electrodes (6a, 6b) of the at least one second pair of electrodes (6a, 6b).

2. Interior aircraft lighting device (2) according to claim 1, comprising:
at least two first pairs of electrodes (51a, 51b, 52a, 52b) and at least two first discharge light modules (3), wherein at least one of the at least two first pairs of electrodes (51a, 51b, 52a, 52b) is assigned to each of the at least two first discharge light modules (3), respectively; and/or
at least two second pairs of electrodes (6a, 6b) and least two second discharge light modules (4), wherein at least one of the at least two second pairs of electrodes (6a, 6b) is assigned to each of the at least two second discharge light modules (4), respectively;
wherein, in particular, the distance (a₁, a₁₁, a₁₂) between the two electrodes (51a, 51b, 52a, 52b) of each of the at least two first pairs of electrodes (51a, 51b, 52a, 52b) is smaller than the distance (a₂) between the two electrodes (6a, 6b) of each of the at least two second pairs of electrodes (6a, 6b).

3. Interior aircraft lighting device (2) according to claim 1 or 2, wherein the distance (a₁, a₁₁, a₁₂) between the two electrodes (5a, 5b, 51a, 51b, 52a, 52b) of the at least one first pair of electrodes (5a, 5b, 51a, 51b, 52a, 52b) is between 2 mm and 7 mm, in particular between 4 mm and 6 mm, more particularly about 5 mm, and/or wherein the second distance (a₂) is between 4 mm and 20 mm, in particular between 8 mm and 12 mm, more particularly about 10 mm.

4. Interior aircraft lighting device (2) according to any of the preceding claims,
wherein the at least two discharge light modules (3, 4) are arranged next to each other in a side-by-side arrangement such that electromagnetic radiation (14), which is emitted by one of the at least two discharge light modules (3, 4), excites the at least one excitable gas in at least one other discharge light module (3, 4);
wherein the at least two discharge light modules (3, 4) are in particular arranged in a parallel configuration, so that the longitudinal directions of the discharge light modules (3, 4) are oriented basically parallel to each other.

5. Interior aircraft lighting device (2) according to any of the preceding claims, wherein the at least two discharge light modules (3, 4) are supported by a common support (7), in particular by a common support plate (7), and/or wherein the interior aircraft lighting device (2) comprises two, three, four, five, six, seven, eight, nine or ten discharge light modules (3, 4).

6. Interior aircraft lighting device (2) according to any of the preceding claims,
wherein at least two pairs of electrodes (51a, 51b, 52a, 52b) are assigned to a particular first discharge light module (3), and/or
wherein at least two pairs of electrodes (6a, 6b) are assigned to a particular second discharge light module (4).

7. Interior aircraft lighting device (2) according to any of the preceding claims, wherein the electromagnetic radiation (14), emitted by the at least one excitable gas, includes electromagnetic radiation (14) in the range of ultraviolet light, in particular electromagnetic radiation (14) in the range from 210 nm to 230 nm, more particularly electromagnetic radiation (14) in the range from 215 nm to 225 nm, and even more particularly electromagnetic radiation (14) in the range from 221 nm to 223 nm.

8. Interior aircraft lighting device (2) according to any of the preceding claims, including an electric power supply for supplying electric power to each pair of electrodes (5a, 5b, 6a, 6b, 51a, 51b, 52a, 52b), wherein the electric power is in particular supplied with a voltage (U) between 1000 V and 5000 V.

9. Aircraft (100), in particular a passenger aircraft (100), comprising at least one interior aircraft lighting device (2) according to any of the preceding claims.

10. Aircraft according to claim 9, comprising a lavatory (108a-108d) and/or a galley (110), wherein at least one interior aircraft lighting device (2) is installed within the lavatory (108a-108d) and/or within the galley (110) of the aircraft (100).

11. Aircraft according to claim 9 or 10, comprising at least one passenger seat (106) and at least one passenger service unit (102) arranged above the at least one passenger seat (106);
wherein at least one interior aircraft lighting device (2) is arranged for irradiating at least a portion of the at least one passenger seat (106) and/or the at least one passenger service unit (102) with electromagnetic radiation (14), which is emitted by the at least two discharge light modules (3, 4) of the at least one interior aircraft lighting device (2).

12. Method of starting an interior aircraft lighting device (2) according to any of
claims 1 to 8 by applying an electric voltage (U) to each pair of electrodes (5a, 5b, 6a, 6b, 51a, 51b, 52a, 52b) of the at least two discharge light modules (3, 4), wherein the electric voltage (U) is in particular an electric voltage (U) between 1000 V and 5000 V.

13. Method of disinfecting at least one component or surface within an aircraft (100), in particular within a passenger aircraft (100), wherein the method includes:
starting an interior aircraft lighting device (2) according to any of claims 1 to 8 for emitting electromagnetic radiation (14) by applying an electric voltage (U) to each pair of electrodes (5a, 5b, 6a, 6b, 51a, 51b, 52a, 52b) of the at least two discharge light modules (3, 4), wherein the electric voltage (U) is in particular an electric voltage (U) between 1000 V and 5000 V; and
irradiating the at least one component or surface with electromagnetic radiation (14) emitted by the at least two discharge light modules (3, 4) of the interior aircraft lighting device (2).

14. Method according to claim 13, wherein the at least one component or surface includes a portion of a passenger seat (106) and/or a passenger service unit (102), and/or wherein the at least one component or surface is located within at least one of a lavatory (108a-108d) and a galley (110) of the aircraft (100).

15. Using an interior aircraft lighting device (2) according to any of claims 1 to 8 for disinfecting at least one component or surface within an aircraft (100) by irradiating the at least one component or surface with electromagnetic radiation (14) emitted by the at least two discharge light modules (3, 4) of the interior aircraft lighting device (2).

## Patentansprüche

1. Luftfahrzeuginnenbeleuchtungsvorrichtung (2), umfassend:
mindestens zwei Entladungsleuchtmodule (3, 4), die mindestens ein erstes Entladungsleuchtmodul (3) und mindestens ein zweites Entladungsleuchtmodul (4) beinhalten, wobei jedes Entladungsleuchtmodul (3, 4) mindestens ein anregbares Gas enthält, das bei elektrischer Anregung elektromagnetische Strahlung (14) emittiert;
mehrere Elektroden (5a, 5b, 6a, 6b, 51a, 51b, 52a, 52b), wobei jedem Entladungsleuchtmodul (3, 4) mindestens ein Paar von Elektroden (5a, 5b, 6a, 6b, 51a, 51b, 52a, 52b) der mehreren Elektroden (5a, 5b, 6a, 6b, 51 a, 51 b, 52a, 52b) zugeordnet ist, um ein elektrisches Feld an das mindestens eine anregbare Gas innerhalb des jeweiligen Entladungsleuchtmoduls (3, 4) anzulegen;
wobei die beiden Elektroden (5a, 5b, 6a, 6b, 51a, 51b, 52a, 52b) jedes Paars von Elektroden (5a, 5b, 6a, 6b, 51a, 51b, 52a, 52b) entlang einer Längsrichtung (A) des jeweiligen Entladungsleuchtmoduls (3, 4) voneinander beabstandet sind;
wobei die Paare von Elektroden (5a, 5b, 6a, 6b, 51a, 51b, 52a, 52b) mindestens ein erstes Paar von Elektroden (5a, 5b, 51a, 51b, 52a, 52b) beinhalten, das dem mindestens einen ersten Entladungsleuchtmodul (3) zugeordnet ist;
**dadurch gekennzeichnet, dass** die Paare von Elektroden (5a, 5b, 6a, 6b, 51 a, 51 b, 52a, 52b) mindestens ein zweites Paar von Elektroden (6a, 6b) beinhalten, das dem mindestens einen zweiten Entladungsleuchtmodul (4) zugeordnet ist; und
wobei ein Abstand (a₁, a₁₁, a₁₂) zwischen den zwei Elektroden (5a, 5b, 51a, 51b, 52a, 52b) des mindestens einen ersten Paars von Elektroden (5a, 5b, 51a, 51b, 52a, 52b) kleiner ist als ein Abstand (a₂) zwischen den zwei Elektroden (6a, 6b) des mindestens einen zweiten Paars von Elektroden (6a, 6b).

2. Luftfahrzeuginnenbeleuchtungsvorrichtung (2) nach Anspruch 1, umfassend:
mindestens zwei erste Paare von Elektroden (51a, 51b, 52a, 52b) und mindestens zwei erste Entladungsleuchtmodule (3), wobei jedem der mindestens zwei ersten Entladungsleuchtmodule (3) jeweils mindestens eines der mindestens zwei ersten Paare von Elektroden (51a, 51b, 52a, 52b) zugeordnet ist; und/oder mindestens zwei zweite Paare von Elektroden (6a, 6b) und mindestens zwei zweite Entladungsleuchtmodule (4), wobei jedem der mindestens zwei zweiten Entladungsleuchtmodule (4) jeweils mindestens eines der mindestens zwei zweiten Paare von Elektroden (6a, 6b) zugeordnet ist;
wobei insbesondere der Abstand (a₁, a₁₁, a₁₂) zwischen den zwei Elektroden (51a, 51b, 52a, 52b) jedes der mindestens zwei ersten Paare von Elektroden (51a, 51b, 52a, 52b) kleiner ist als der Abstand (a₂) zwischen den zwei Elektroden (6a, 6b) jedes der mindestens zwei zweiten Paare von Elektroden (6a, 6b) .

3. Luftfahrzeuginnenbeleuchtungsvorrichtung (2) nach Anspruch 1 oder 2, wobei der Abstand (a₁, a₁₁, a₁₂) zwischen den zwei Elektroden (5a, 5b, 51a, 51b, 52a, 52b) des mindestens einen ersten Paars von Elektroden (5a, 5b, 51a, 51b, 52a, 52b) zwischen 2 mm und 7 mm, insbesondere zwischen 4 mm und 6 mm, weiter insbesondere etwa 5 mm beträgt, und/oder wobei der zweite Abstand (a₂) zwischen 4 mm und 20 mm, insbesondere zwischen 8 mm und 12 mm, weiter insbesondere etwa 10 mm beträgt.

4. Luftfahrzeuginnenbeleuchtungsvorrichtung (2) nach einem der vorhergehenden Ansprüche,
wobei die mindestens zwei Entladungsleuchtmodule (3, 4) in einer Seite-an-Seite-Anordnung nebeneinander angeordnet sind, so dass elektromagnetische Strahlung (14), die von einem der mindestens zwei Entladungsleuchtmodule (3, 4) emittiert wird, das mindestens eine anregbare Gas in mindestens einem anderen Entladungsleuchtmodul (3, 4) anregt;
wobei die mindestens zwei Entladungsleuchtmodule (3, 4) insbesondere in einer Parallelkonfiguration angeordnet sind, so dass die Längsrichtungen der Entladungsleuchtmodule (3, 4) im Wesentlichen parallel zueinander ausgerichtet sind.

5. Luftfahrzeuginnenbeleuchtungsvorrichtung (2) nach einem der vorhergehenden Ansprüche,
wobei die mindestens zwei Entladungsleuchtmodule (3, 4) durch einen gemeinsamen Träger (7), insbesondere durch eine gemeinsame Trägerplatte (7), getragen werden, und/oder wobei die Luftfahrzeuginnenbeleuchtungsvorrichtung (2) zwei, drei, vier, fünf, sechs, sieben, acht, neun oder zehn Entladungsleuchtmodule (3, 4) umfasst.

6. Luftfahrzeuginnenbeleuchtungsvorrichtung (2) nach einem der vorhergehenden Ansprüche,
wobei mindestens zwei Paare von Elektroden (51a, 51b, 52a, 52b) einem bestimmten ersten Entladungsleuchtmodul (3) zugeordnet sind, und/oder
wobei mindestens zwei Paare von Elektroden (6a, 6b) einem bestimmten zweiten Entladungsleuchtmodul (4) zugeordnet sind.

7. Luftfahrzeuginnenbeleuchtungsvorrichtung (2) nach einem der vorhergehenden Ansprüche,
wobei die von dem mindestens einen anregbaren Gas emittierte elektromagnetische Strahlung (14) elektromagnetische Strahlung (14) im Bereich des ultravioletten Lichts, insbesondere elektromagnetische Strahlung (14) im Bereich von 210 nm bis 230 nm, weiter insbesondere elektromagnetische Strahlung (14) im Bereich von 215 nm bis 225 nm, und noch weiter insbesondere elektromagnetische Strahlung (14) im Bereich von 221 nm bis 223 nm beinhaltet.

8. Luftfahrzeuginnenbeleuchtungsvorrichtung (2) nach einem der vorhergehenden Ansprüche,
beinhaltend eine elektrische Leistungsversorgung zur Versorgung jedes Paars von Elektroden (5a, 5b, 6a, 6b, 51a, 51b, 52a, 52b) mit elektrischer Leistung, wobei die elektrische Leistung insbesondere mit einer Spannung (U) zwischen 1000 V und 5000 V bereitgestellt wird.

9. Luftfahrzeug (100), insbesondere ein Passagierluftfahrzeug (100), umfassend mindestens eine Luftfahrzeuginnenbeleuchtungsvorrichtung (2) nach einem der vorhergehenden Ansprüche.

10. Luftfahrzeug nach Anspruch 9, umfassend eine Toilette (108a-108d) und/oder eine Bordküche (110), wobei mindestens eine Luftfahrzeuginnenbeleuchtungsvorrichtung (2) innerhalb der Toilette (108a-108d) und/oder innerhalb der Bordküche (110) des Luftfahrzeugs (100) installiert ist.

11. Luftfahrzeug nach Anspruch 9 oder 10, umfassend mindestens einen Passagiersitz (106) und mindestens eine über dem mindestens einen Passagiersitz (106) angeordnete Passagierserviceeinheit (102);
wobei mindestens eine Luftfahrzeuginnenbeleuchtungsvorrichtung (2) zum Bestrahlen mindestens eines Teils des mindestens einen Passagiersitzes (106) und/oder der mindestens einen Passagierserviceeinheit (102) mit elektromagnetischer Strahlung (14) angeordnet ist, die von den mindestens zwei Entladungsleuchtmodulen (3, 4) der mindestens einen Luftfahrzeuginnenbeleuchtungsvorrichtung (2) emittiert wird.

12. Verfahren zum Starten einer Luftfahrzeuginnenbeleuchtungsvorrichtung (2) nach einem der Ansprüche 1 bis 8 durch Anlegen einer elektrischen Spannung (U) an jedes Paar von Elektroden (5a, 5b, 6a, 6b, 51a, 51b, 52a, 52b) der mindestens zwei Entladungsleuchtmodule (3, 4), wobei die elektrische Spannung (U) insbesondere eine elektrische Spannung (U) zwischen 1000 V und 5000 V ist.

13. Verfahren zum Desinfizieren von mindestens einer Komponente oder Oberfläche innerhalb eines Luftfahrzeugs (100), insbesondere innerhalb eines Passagierluftfahrzeugs (100), wobei das Verfahren umfasst:
Starten einer Luftfahrzeuginnenbeleuchtungsvorrichtung (2) nach einem der Ansprüche 1 bis 8 zum Emittieren von elektromagnetischer Strahlung (14) durch Anlegen einer elektrischen Spannung (U) an jedes Paar von Elektroden (5a, 5b, 6a, 6b, 51a, 51b, 52a, 52b) der mindestens zwei Entladungsleuchtmodule (3, 4), wobei die elektrische Spannung (U) insbesondere eine elektrische Spannung (U) zwischen 1000 V und 5000 V ist; und
Bestrahlen der mindestens einen Komponente oder Oberfläche mit von den mindestens zwei Entladungsleuchtmodulen (3, 4) der Luftfahrzeuginnenbeleuchtungsvorrichtung (2) emittierter elektromagnetischer Strahlung (14).

14. Verfahren nach Anspruch 13, wobei die mindestens eine Komponente oder Oberfläche einen Teil eines Passagiersitzes (106) und/oder einer Passagierserviceeinheit (102) beinhaltet und/oder wobei die mindestens eine Komponente oder Oberfläche innerhalb mindestens einer von einer Toilette (108a-108d) und einer Bordküche (110) des Luftfahrzeugs (100) angeordnet ist.

15. Verwendung einer Luftfahrzeuginnenbeleuchtungsvorrichtung (2) nach einem der Ansprüche 1 bis 8 zum Desinfizieren mindestens einer Komponente oder Oberfläche innerhalb eines Luftfahrzeugs (100) durch Bestrahlen der mindestens einen Komponente oder Oberfläche mit von den mindestens zwei Entladungsleuchtmodulen (3, 4) der Luftfahrzeuginnenbeleuchtungsvorrichtung (2) emittierter elektromagnetischer Strahlung (14).

## Revendications

1. Dispositif d'éclairage intérieur d'aéronef (2), comprenant :
au moins deux modules de lumière à décharge (3, 4), comportant au moins un premier module de lumière à décharge (3) et au moins un second module de lumière à décharge (4), dans lequel chaque module de lumière à décharge (3, 4) contient au moins un gaz excitable qui émet un rayonnement électromagnétique (14) sous l'effet d'une excitation électrique ;
de multiples électrodes (5a, 5b, 6a, 6b, 51a, 51b, 52a, 52b), dans lequel au moins une paire d'électrodes (5a, 5b, 6a, 6b, 51a, 51b, 52a, 52b) des multiples électrodes (5a, 5b, 6a, 6b, 51a, 51b, 52a, 52b) est attribuée à chaque module de lumière à décharge (3, 4) pour appliquer un champ électrique à l'au moins un gaz excitable à l'intérieur du module de lumière à décharge (3, 4) respectif ;
dans lequel les deux électrodes (5a, 5b, 6a, 6b, 51a, 51b, 52a, 52b) de chaque paire d'électrodes (5a, 5b, 6a, 6b, 51a, 51b, 52a, 52b) sont espacées l'une de l'autre le long d'une direction longitudinale (A) du module de lumière à décharge (3, 4) respectif ;
dans lequel les paires d'électrodes (5a, 5b, 6a, 6b, 51a, 51b, 52a, 52b) comportent au moins une première paire d'électrodes (5a, 5b, 51a, 51b, 52a, 52b), qui est attribuée à l'au moins un premier module de lumière à décharge (3) ;
**caractérisé en ce que** les paires d'électrodes (5a, 5b, 6a, 6b, 51a, 51b, 52a, 52b) comportent au moins une seconde paire d'électrodes (6a, 6b), qui est attribuée à l'au moins un second module de lumière à décharge (4) ; et
dans lequel une distance (a₁, an, a₁₂) entre les deux électrodes (5a, 5b, 51a, 51b, 52a, 52b) de l'au moins une première paire d'électrodes (5a, 5b, 51a, 51b, 52a, 52b) est inférieure à une distance (a₂) entre les deux électrodes (6a, 6b) de l'au moins une seconde paire d'électrodes (6a, 6b).

2. Dispositif d'éclairage intérieur d'aéronef (2) selon la revendication 1, comprenant :
au moins deux premières paires d'électrodes (51a, 51b, 52a, 52b) et au moins deux premiers modules de lumière à décharge (3), dans lequel au moins l'une des au moins deux premières paires d'électrodes (51a, 51b, 52a, 52b) est attribuée respectivement à chacun des au moins deux premiers modules de lumière à décharge (3) ; et/ou
au moins deux secondes paires d'électrodes (6a, 6b) et au moins deux seconds modules de lumière à décharge (4), dans lequel au moins l'une des au moins deux secondes paires d'électrodes (6a, 6b) est attribuée respectivement à chacun des au moins deux seconds modules de lumière à décharge (4) ;
dans lequel, en particulier, la distance (a₁, a₁₁, a₁₂) entre les deux électrodes (51a, 51b, 52a, 52b) de chacune des au moins deux premières paires d'électrodes (51a, 51b, 52a, 52b) est inférieure à la distance (a₂) entre les deux électrodes (6a, 6b) de chacune des au moins deux secondes paires d'électrodes (6a, 6b) .

3. Dispositif d'éclairage intérieur d'aéronef (2) selon la revendication 1 ou 2, dans lequel la distance (a₁, a₁₁, a₁₂) entre les deux électrodes (5a, 5b, 51a, 51b, 52a, 52b) de l'au moins une première paire d'électrodes (5a, 5b, 51a, 51b, 52a, 52b) est comprise entre 2 mm et 7 mm, en particulier entre 4 mm et 6 mm, plus particulièrement environ 5 mm, et/ou dans lequel la seconde distance (a₂) est comprise entre 4 mm et 20 mm, en particulier entre 8 mm et 12 mm, plus particulièrement environ 10 mm.

4. Dispositif d'éclairage intérieur d'aéronef (2) selon l'une quelconque des revendications précédentes,
dans lequel les au moins deux modules de lumière à décharge (3, 4) sont disposés l'un à côté de l'autre dans une disposition côte à côte de telle sorte qu'un rayonnement électromagnétique (14), qui est émis par l'un des au moins deux modules de lumière à décharge (3, 4), excite l'au moins un gaz excitable dans au moins un autre module de lumière à décharge (3, 4) ;
dans lequel les au moins deux modules de lumière à décharge (3, 4) sont en particulier disposés dans une configuration parallèle, de sorte que les directions longitudinales des modules de lumière à décharge (3, 4) sont orientées essentiellement parallèlement les unes aux autres.

5. Dispositif d'éclairage intérieur d'aéronef (2) selon l'une quelconque des revendications précédentes,
dans lequel les au moins deux modules de lumière à décharge (3, 4) sont supportés par un support commun (7), en particulier par une plaque de support commune (7), et/ou
dans lequel le dispositif d'éclairage intérieur d'aéronef (2) comprend deux, trois, quatre, cinq, six, sept, huit, neuf ou dix modules de lumière à décharge (3, 4).

6. Dispositif d'éclairage intérieur d'aéronef (2) selon l'une quelconque des revendications précédentes,
dans lequel au moins deux paires d'électrodes (51a, 51b, 52a, 52b) sont attribuées à un premier module de lumière à décharge (3) particulier, et/ou
dans lequel au moins deux paires d'électrodes (6a, 6b) sont attribuées à un second module de lumière à décharge (4) particulier.

7. Dispositif d'éclairage intérieur d'aéronef (2) selon l'une quelconque des revendications précédentes,
dans lequel le rayonnement électromagnétique (14) émis par l'au moins un gaz excitable comporte un rayonnement électromagnétique (14) dans la plage de la lumière ultraviolette, en particulier un rayonnement électromagnétique (14) dans la plage de 210 nm à 230 nm, plus particulièrement un rayonnement électromagnétique (14) dans la plage de 215 nm à 225 nm, et encore plus particulièrement un rayonnement électromagnétique (14) dans la plage de 221 nm à 223 nm.

8. Dispositif d'éclairage intérieur d'aéronef (2) selon l'une quelconque des revendications précédentes,
comportant une alimentation électrique pour fournir de l'énergie électrique à chaque paire d'électrodes (5a, 5b, 6a, 6b, 51a, 51b, 52a, 52b), dans lequel l'énergie électrique est en particulier fournie avec une tension (U) comprise entre 1 000 V et 5 000 V.

9. Aéronef (100), en particulier avion commercial (100), comprenant au moins un dispositif d'éclairage intérieur d'aéronef (2) selon l'une quelconque des revendications précédentes.

10. Aéronef selon la revendication 9, comprenant des toilettes (108a à 108d) et/ou une cuisine (110), dans lequel au moins un dispositif d'éclairage intérieur d'aéronef (2) est installé à l'intérieur des toilettes (108a à 108d) et/ou à l'intérieur de la cuisine (110) de l'aéronef (100).

11. Aéronef selon la revendication 9 ou 10, comprenant au moins un siège passager (106) et au moins une unité de service aux passagers (102) disposée au-dessus de l'au moins un siège passager (106) ;
dans lequel au moins un dispositif d'éclairage intérieur d'aéronef (2) est disposé pour irradier au moins une partie de l'au moins un siège passager (106) et/ou de l'au moins une unité de service aux passagers (102) avec un rayonnement électromagnétique (14), qui est émis par les au moins deux modules de lumière à décharge (3, 4) de l'au moins un dispositif d'éclairage intérieur d'aéronef (2).

12. Procédé de démarrage d'un dispositif d'éclairage intérieur d'aéronef (2) selon l'une quelconque des revendications 1 à 8 en appliquant une tension électrique (U) à chaque paire d'électrodes (5a, 5b, 6a, 6b, 51a, 51b, 52a, 52b) des au moins deux modules de lumière à décharge (3, 4), dans lequel la tension électrique (U) est en particulier une tension électrique (U) comprise entre 1 000 V et 5 000 V.

13. Procédé de désinfection d'au moins un composant ou une surface à l'intérieur d'un aéronef (100), en particulier à l'intérieur d'un avion commercial (100), dans lequel le procédé comporte :
le démarrage d'un dispositif d'éclairage intérieur d'aéronef (2) selon l'une quelconque des revendications 1 à 8 pour émettre un rayonnement électromagnétique (14) en appliquant une tension électrique (U) à chaque paire d'électrodes (5a, 5b, 6a, 6b, 51a, 51b, 52a, 52b) des au moins deux modules de lumière à décharge (3, 4), dans lequel la tension électrique (U) est en particulier une tension électrique (U) comprise entre 1 000 V et 5 000 V ; et
l'irradiation de l'au moins un composant ou une surface avec un rayonnement électromagnétique (14) émis par les au moins deux modules de lumière à décharge (3, 4) du dispositif d'éclairage intérieur d'aéronef (2).

14. Procédé selon la revendication 13, dans lequel l'au moins un composant ou une surface comporte une partie d'un siège passager (106) et/ou d'une unité de service aux passagers (102), et/ou dans lequel l'au moins un composant ou une surface est situé à l'intérieur d'au moins l'un des toilettes (108a à 108d) et d'une cuisine (110) de l'aéronef (100).

15. Utilisation d'un dispositif d'éclairage intérieur d'aéronef (2) selon l'une quelconque des revendications 1 à 8 pour désinfecter au moins un composant ou une surface à l'intérieur d'un aéronef (100) en irradiant l'au moins un composant ou une surface avec un rayonnement électromagnétique (14) émis par les au moins deux modules de lumière à décharge (3, 4) du dispositif d'éclairage intérieur d'aéronef (2).
